# EUROPEAN PATENT APPLICATION

(11) **EP 4 825 369 A1**
(43) Date of publication of application: **21.10.2026**
(21) Application number: 26168690.1
(22) Date of filing: 27.03.2026
(51) Int. Cl.: A61K 8/49, A61Q 19/00, C07D 233/88

(54) **ALLANTOIN MICRO-NEEDLE CRYSTAL AND COSMETIC COMPOSITION CONTAINING THE SAME**

(30) Priority: 14.04.2025 KR 20250048263
(71) Applicant: Amorepacific Corporation, Seoul 04386 (KR)
(72) Inventor: Kim, Suil, 17074 Yongin-si, Gyeonggi-do (KR); Jang, Geunhyuk, 17074 Yongin-si, Gyeonggi-do (KR); Park, Sodam, 17074 Yongin-si, Gyeonggi-do (KR); Park, Seunghan, 17074 Yongin-si, Gyeonggi-do (KR); Baek, Heungsoo, 17074 Yongin-si, Gyeonggi-do (KR); You, Jaewon, 17074 Yongin-si, Gyeonggi-do (KR); Lee, Eun Soo, 17074 Yongin-si, Gyeonggi-do (KR)
(74) Representative: Schön, Christoph

(57) **Abstract**

The present specification relates to an allantoin micro-needle crystal, and a cosmetic composition and an active ingredient delivery system comprising the same, wherein the allantoin micro-needle crystal according to one aspect of the present invention is one in which poorly soluble allantoin is crystallized to have excellent solubility and thus can be utilized as a component of a cosmetic composition, and by having an acicular shape like a needle with an average length of a long axis of 5 to 500 µm, it can penetrate into skin and be absorbed into the skin simply by applying it to the skin, and has an excellent effect of being dissolved in the skin by rolling after application to have excellent safety, and being easily stored and distributed by being stably maintained without a change in physical properties even under harsh conditions.

## Description

### BACKGROUND OF THE INVENTION

### Field of the Invention

The present specification relates to an allantoin micro-needle crystal and a cosmetic composition comprising the same.

### Description of the Related Art

Recently, home-care skin management cosmetics utilizing a "micro-needle (fine needle)" technology, which is mainly utilized in dermatological beauty medical devices, are gaining popularity. Such cosmetics have established a selling point with an expectation of being able to experience efficacy obtainable through dermatological procedures by applying a dermatological procedure principle that provides a stinging and uncomfortable sensation. A core ingredient of most home-care skin management cosmetics utilizing the micro-needle technology is a micro-needle in which cosmetic ingredients such as a *Centella asiatica* extract are contained in a purified silica needle composed of animal-based raw materials, and it serves as a physical delivery system that physically helps skin penetration without a separate device, thereby inducing a purchase motivation in consumers, enabling intuitive experience of active ingredients, and serving to help absorption of other active ingredients.

However, spicule (hydrolyzed sponge), which is a main component of such a micro-needle, is an animal-based raw material and has a skeleton derived from a sponge, and thus about 99% is composed of silica. Since such spicules are insoluble components, they are too stinging to be used on sensitive skin after being applied to the skin, and there is a problem that pain continues to remain over time as needles are stuck in the skin.

Accordingly, the present inventors performed research on a new material capable of exhibiting an effect similar to receiving a dermatological procedure by having a shape like a micro-needle and thus enabling skin penetration, while being able to solve disadvantages of the spicule by being dissolved in skin, and as a result, completed the present invention.

### SUMMARY OF THE INVENTION

The present inventors performed research on a material that has a shape like a micro-needle to enable skin penetration and thus exhibits an effect similar to receiving a dermatological procedure, while being dissolved in skin, and as a result, confirmed that a crystal in which atypical allantoin is precipitated in a shape like a micro-needle can obtain such effects.

Therefore, in one aspect, an object of the present invention is to provide a new material that has a shape like a micro-needle while providing a user with a sensation like receiving a dermatological procedure when applied to skin and is safe by dissolving after penetrating into the skin.

One aspect of the present invention provides an allantoin micro-needle crystal having an average length of a long axis of 5 to 500 µm.

Another aspect of the present invention provides a cosmetic composition comprising the allantoin micro-needle crystal as an active ingredient.

The allantoin micro-needle crystal according to one aspect of the present invention is one in which poorly soluble allantoin is crystallized to have excellent solubility and thus can be utilized as a component of a cosmetic composition, and by having an acicular shape like a needle with an average length of a long axis of 5 to 500 µm, it can penetrate into skin and be absorbed into the skin simply by applying it to the skin, and has an excellent effect of being dissolved in the skin by rolling after application to have excellent safety, and being easily stored and distributed by being stably maintained without a change in physical properties even under harsh conditions.

### BRIEF DESCRIPTION OF THE DRAWINGS

FIG. 1 is a photograph of an allantoin micro-needle crystal according to an embodiment of the present invention.
FIG. 2 is a photograph taken when a general allantoin powder is dissolved in deionized water.
FIG. 3 is a photograph taken when an allantoin micro-needle crystal according to an embodiment of the present invention is dissolved in deionized water.
FIG. 4A is a photograph of a cross-section of an untreated group to which nothing was treated after Nile red staining in an *ex vivo* stratum corneum residual evaluation experiment according to an embodiment of the present invention.
FIG. 4B is a photograph of a cross-section when 18 hours have elapsed after an allantoin powder, which is a control group, was applied to a skin biopsy tissue in the *ex vivo* stratum corneum residual evaluation experiment according to an embodiment of the present invention.
FIG. 4C is a photograph of a cross-section when 18 hours have elapsed after the allantoin micro-needle crystal according to an embodiment of the present invention was applied to a skin biopsy tissue in the *ex vivo* stratum corneum residual evaluation experiment according to an embodiment of the present invention.
FIG. 5 is a diagram showing a degree of sensation perception according to a content of the allantoin micro-needle crystal according to an embodiment of the present invention.
FIG. 6 is a photograph taken to confirm formulation stability when the allantoin micro-needle crystal according to an embodiment of the present invention is stored under a room temperature (RT) condition, a refrigerated condition, a 37°C condition, a 45°C condition, and a cycling (CYC) condition, respectively.

### DETAILED DESCRIPTION OF THE INVENTION

In this specification, "acicular" or "needle" refers to a shape that is thin like a needle and has a sharp tip, and "crystal" refers to a crystalline solid that does not substantially include a fixed molar ratio of solvent molecules within a crystal lattice, that is, a crystalline solid that is not a solvate, wherein "acicular crystal" or "needle crystal" refers to a needle-shaped crystal.

Hereinafter, the present invention will be described in detail.

In one aspect, the present invention provides an allantoin micro-needle crystal having an average length of a long axis of 5 to 500 µm.

Allantoin (C₄H₆N₄O₃, molecular weight 158.116 g/mol, IUPAC name (2,5-dioxo-4-imidazolidinyl) urea, CAS No. 97-59-6, EC No. 202-592-8) according to one aspect of the present invention is an ingredient approved by the U.S. FDA as an over-the-counter (OTC) drug as an active ingredient for skin protection, and is known as a poorly soluble ingredient. The allantoin may be represented by the following [Chemical Formula 1].

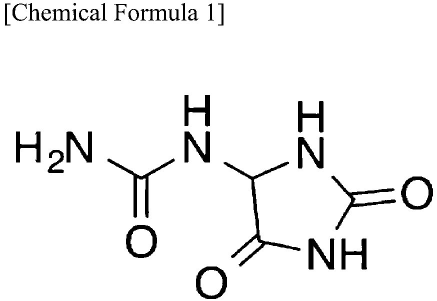

The allantoin crystal according to one aspect of the present invention has a micro-needle structure and thus may give a fine stimulus to skin when applied to the skin, thereby promoting absorption into the skin.

The average length of the long axis of the allantoin micro-needle crystal according to one aspect of the present invention may be 5 to 500 µm. Specifically, the average length of the long axis of the allantoin micro-needle crystal may be 5 µm or more, 10 µm or more, 20 µm or more, 30 µm or more, 40 µm or more, 50 µm or more, 55 µm or more, 60 µm or more, 65 µm or more, 70 µm or more, 75 µm or more, 80 µm or more, 85 µm or more, 90 µm or more, 92 µm or more, 94 µm or more, 95 µm or more, 96 µm or more, 98 µm or more, 100 µm or more, 110 µm or more, 120 µm or more, 130 µm or more, 140 µm or more, 150 µm or more, 160 µm or more, 170 µm or more, 180 µm or more, 190 µm or more, 200 µm or more, 250 µm or more, 300 µm or more, 350 µm or more, 400 µm or more, or 450 µm or more, and may be 500 µm or less, 450 µm or less, 400 µm or less, 350 µm or less, 300 µm or less, 250 µm or less, 240 µm or less, 230 µm or less, 225 µm or less, 220 µm or less, 218 µm or less, 216 µm or less, 214 µm or less, 212 µm or less, 210 µm or less, 208 µm or less, 206 µm or less, 204 µm or less, 202 µm or less, 200 µm or less, 180 µm or less, 160 µm or less, 140 µm or less, 120 µm or less, 100 µm or less, 90 µm or less, 80 µm or less, 70 µm or less, or 60 µm or less. More specifically, the average length of the long axis of the allantoin micro-needle crystal may be 100 to 200 µm, and even more specifically, may be 150 to 200 µm.

The thickness of the allantoin micro-needle crystal according to one aspect of the present invention may be greater than 0 µm and 40 µm or less. Specifically, the thickness of the allantoin micro-needle crystal may be greater than 0 µm, 2 µm or more, 4 µm or more, 5 µm or more, 6 µm or more, 7 µm or more, 8 µm or more, 9 µm or more, 10 µm or more, 11 µm or more, 12 µm or more, 13 µm or more, 14 µm or more, 15 µm or more, 16 µm or more, 17 µm or more, 18 µm or more, 19 µm or more, 20 µm or more, 22 µm or more, 24 µm or more, 26 µm or more, 28 µm or more, 30 µm or more, or 35 µm or more, and may be 40 µm or less, 38 µm or less, 36 µm or less, 34 µm or less, 32 µm or less, 30 µm or less, 29 µm or less, 28 µm or less, 27 µm or less, 26 µm or less, 25 µm or less, 24 µm or less, 23 µm or less, 22 µm or less, 21 µm or less, 20 µm or less, 15 µm or less, 10 µm or less, or 8 µm or less. More specifically, the thickness of the allantoin micro-needle crystal may be 10 to 20 µm. The allantoin micro-needle crystal according to one aspect of the present invention has a thickness of greater than 0 µm and 40 µm or less, and thus may slightly penetrate a skin surface and pass through a stratum corneum.

The allantoin micro-needle crystal according to one aspect of the present invention may be one crystallized by precipitating atypical allantoin through an anti-solvent crystallization method.

The allantoin micro-needle crystal according to one aspect of the present invention may be soluble. The allantoin micro-needle crystal may be water-soluble or oil-soluble. According to an embodiment of the present invention, the allantoin micro-needle crystal according to one aspect of the present invention has an excellent solubility in a solvent and thus has a soluble characteristic, which has an effect of solving a problem of poor solubility of general allantoin powder (see Manufacturing Examples and Tables 1 and 2).

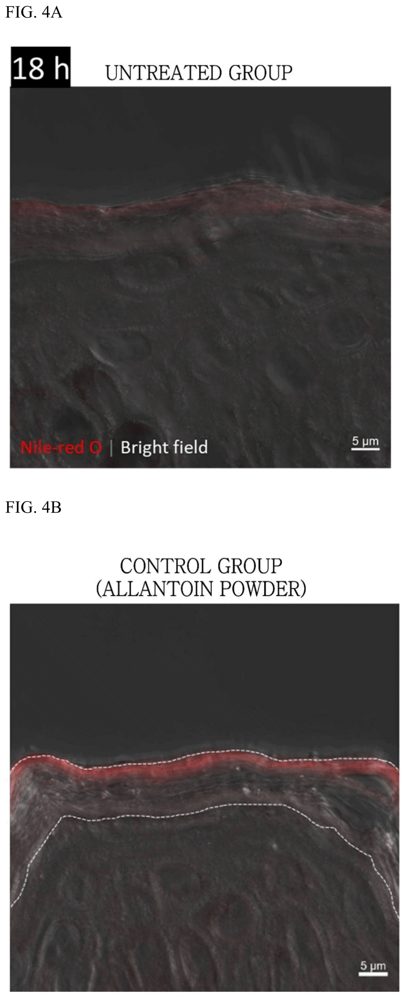

The allantoin micro-needle crystal according to one aspect of the present invention may be one that penetrates into skin when applied to the skin. In addition, the allantoin micro-needle crystal according to one aspect of the present invention may exist inside a skin stratum corneum when 2 to 36 hours have elapsed after being applied to the skin. Specifically, the allantoin micro-needle crystal may exist inside a skin stratum corneum when 2 hours or more, 3 hours or more, 4 hours or more, 5 hours or more, 6 hours or more, 7 hours or more, 8 hours or more, 9 hours or more, 10 hours or more, 12 hours or more, 14 hours or more, 16 hours or more, 18 hours or more, 20 hours or more, 24 hours or more, 30 hours or more, or 36 hours or more have elapsed after being applied to the skin. According to an embodiment of the present invention, by having a micro-needle shape like a needle, it remains inside the stratum corneum even after much time has elapsed after being applied to the skin, and thus, simply by applying it to the skin, it may penetrate into the skin and be absorbed into the skin (see Experimental Example 1 and FIG. 4B).

The allantoin micro-needle crystal according to one aspect of the present invention may be dissolved in skin and absorbed into the skin after being applied to the skin. The process may be as follows: first, when applying the allantoin micro-needle crystal onto skin, the allantoin micro-needle crystal itself may be attached to a skin surface through direct application. Then, the applied allantoin micro-needle crystal has a thickness of greater than 0 µm and 40 µm or less, specifically 5 to 40 µm, and thus slightly penetrates a skin surface and may pass through a skin stratum corneum. In this case, an allantoin ingredient may be immediately delivered into the skin, and due to the micro-needle structure, a fine stimulus is applied to the skin, so that absorption into the skin can be further promoted. Thereafter, when rolling or rubbing the skin surface with a hand or a finger about 10 times, contact of allantoin fine crystals with the skin surface is maximized, which can help penetration to be smoothly performed. The rolling or rubbing motion may promote dissolution of the allantoin micro-needle crystal in the skin and absorption into the skin, and a micro-circulation improvement effect by fine stimulation may also be expected. Further, the allantoin micro-needle crystal penetrated into the skin or the skin stratum corneum is slowly dissolved while contacting with moisture in the skin, and allantoin is released, which may be performed within 2 to 36 hours after application. A specific absorption process is first absorbed into a stratum corneum (Keratin Layer), which reacts with subcutaneous moisture to be gradually dissolved, and some ingredients gradually reach even an epidermis layer (Epidermis) to be secondarily absorbed. In general, the allantoin micro-needle crystal is mostly dissolved and absorbed within the stratum corneum.

The allantoin micro-needle crystal according to one aspect of the present invention may be dissolved and absorbed in skin when repeatedly rolled 10 times with a weight of 0.5 to 1.0 kg/cm² after being applied to the skin. The allantoin micro-needle crystal may be dissolved and absorbed in skin when 2 hours or more, 3 hours or more, 4 hours or more, 5 hours or more, 6 hours or more, 7 hours or more, 8 hours or more, 9 hours or more, 12 hours or more, 15 hours or more, 18 hours or more, 21 hours or more, 24 hours or more, 27 hours or more, 30 hours or more, 33 hours or more, or 36 hours or more have elapsed after being applied to the skin, but dissolution and absorption into the skin may be promoted by rolling with a hand or a finger. Specifically, the allantoin micro-needle crystal may be dissolved in skin and absorbed into a skin stratum corneum when repeatedly rolled 1 time, 2 times, 3 times, 4 times, 5 times, 6 times, 7 times, 8 times, 9 times, or 10 times with a weight of 0.5 kg/cm² or more, 0.6 kg/cm² or more, 0.7 kg/cm² or more, 0.8 kg/cm² or more, or 0.9 kg/cm² or more, and a weight of 1.0 kg/cm² or less, 0.9 kg/cm² or less, 0.8 kg/cm² or less, 0.7 kg/cm² or less, or 0.6 kg/cm² or less after being applied to the skin. The rolling or rubbing motion promotes dissolution of the allantoin micro-needle crystal and its absorption into the skin, and a micro-circulation improvement effect by fine stimulation may also be expected.

The allantoin micro-needle crystal according to one aspect of the present invention may comprise 3 to 16 wt% of the allantoin based on a total weight of the allantoin micro-needle crystal. Specifically, the allantoin micro-needle crystal may comprise 3 wt% or more, 3.5 wt% or more, 4 wt% or more, 4.2 wt% or more, 4.4 wt% or more, 4.6 wt% or more, 4.8 wt% or more, 5 wt% or more, 5.2 wt% or more, 5.4 wt% or more, 5.6 wt% or more, 5.8 wt% or more, 6 wt% or more, 7 wt% or more, 8 wt% or more, 9 wt% or more, 10 wt% or more, 11 wt% or more, 12 wt% or more, or 14 wt% or more of the allantoin based on the total weight of the allantoin micro-needle crystal, and may comprise 16 wt% or less, 15 wt% or less, 14 wt% or less, 13 wt% or less, 12 wt% or less, 11 wt% or less, 10 wt% or less, 9 wt% or less, 8.8 wt% or less, 8.6 wt% or less, 8.4 wt% or less, 8.2 wt% or less, 8 wt% or less, 7.8 wt% or less, 7.6 wt% or less, 7.4 wt% or less, 7.2 wt% or less, 7 wt% or less, 6 wt% or less, 5 wt% or less, or 4 wt% or less of the allantoin based on the total weight of the allantoin micro-needle crystal. More specifically, the allantoin micro-needle crystal may comprise 4 to 9 wt% of the allantoin based on the total weight of the allantoin micro-needle crystal.

The allantoin micro-needle crystal according to one aspect of the present invention may further comprise betaine. The allantoin micro-needle crystal may crystallize allantoin in a micro-needle shape with higher purity and yield when comprising betaine.

The allantoin micro-needle crystal according to one aspect of the present invention may comprise 9 to 72 wt% of the betaine based on the total weight of the allantoin micro-needle crystal. Specifically, the allantoin micro-needle crystal may comprise 9 wt% or more, 10 wt% or more, 12 wt% or more, 14 wt% or more, 16 wt% or more, 18 wt% or more, 20 wt% or more, 22 wt% or more, 24 wt% or more, 26 wt% or more, 28 wt% or more, 30 wt% or more, 35 wt% or more, 36 wt% or more, 40 wt% or more, 50 wt% or more, or 60 wt% or more of the betaine based on the total weight of the allantoin micro-needle crystal, and may comprise 72 wt% or less, 70 wt% or less, 60 wt% or less, 50 wt% or less, 40 wt% or less, 38 wt% or less, 36 wt% or less, 34 wt% or less, 32 wt% or less, 30 wt% or less, 28 wt% or less, 26 wt% or less, 24 wt% or less, 22 wt% or less, 20 wt% or less, 19 wt% or less, 18 wt% or less, 16 wt% or less, 14 wt% or less, 12 wt% or less, or 10 wt% or less of the betaine based on the total weight of the allantoin micro-needle crystal. More specifically, the allantoin micro-needle crystal may comprise 12 to 36 wt% of the betaine based on the total weight of the allantoin micro-needle crystal.

In another aspect, the present invention provides a cosmetic composition comprising an allantoin micro-needle crystal as an active ingredient. The description of the allantoin micro-needle crystal also applies to the cosmetic composition.

A cosmetic composition according to one aspect of the present invention may comprise 3 to 12 wt% of the allantoin micro-needle crystal based on a total weight of the cosmetic composition. Specifically, the cosmetic composition may comprise 3 wt% or more, 3.5 wt% or more, 4 wt% or more, 4.2 wt% or more, 4.4 wt% or more, 4.6 wt% or more, 4.8 wt% or more, 5 wt% or more, 5.2 wt% or more, 5.4 wt% or more, 5.6 wt% or more, 5.8 wt% or more, 6 wt% or more, 7 wt% or more, 8 wt% or more, 9 wt% or more, or 10 wt% or more of the allantoin micro-needle crystal based on the total weight of the composition, and may comprise 12 wt% or less, 11 wt% or less, 10 wt% or less, 9 wt% or less, 8.8 wt% or less, 8.6 wt% or less, 8.4 wt% or less, 8.2 wt% or less, 8 wt% or less, 7.8 wt% or less, 7.6 wt% or less, 7.4 wt% or less, 7.2 wt% or less, 7 wt% or less, 6 wt% or less, 5 wt% or less, or 4 wt% or less of the allantoin micro-needle crystal based on the total weight of the composition. More specifically, the cosmetic composition may comprise 4 to 9 wt% of the allantoin micro-needle crystal based on a total weight of the composition.

The cosmetic composition according to one aspect of the present invention may further comprise betaine. The allantoin micro-needle crystal may crystallize allantoin in a micro-needle shape with higher purity and yield when comprising betaine.

The cosmetic composition according to one aspect of the present invention may be for exfoliation, skin moisturizing, skin wound healing, promoting skin regeneration, or skin protection against irritation.

In the composition according to one aspect of the present invention, an appearance of the cosmetic composition may contain a cosmetically or dermatologically acceptable medium or base. This includes all formulations suitable for topical application, and may be provided, for example, in a form of a solution, a gel, a solid, a paste anhydrous product, an emulsion obtained by dispersing an oil phase in an aqueous phase, a suspension, a microemulsion, a microcapsule, a microgranule, or an ionic (liposome) and non-ionic vesicular dispersant, or in a form of a cream, a toner, a lotion, a powder, an ointment, a spray, or a concealer stick. These compositions may be manufactured according to conventional methods in the art. The composition according to the present disclosure may also be used in the form of a foam, or an aerosol composition further containing a compressed propellant.

The cosmetic composition according to one aspect of the present invention is not particularly limited in its formulation, and may be formulated into cosmetics such as, for example, softening cosmetic water, astringent cosmetic water, nourishing cosmetic water, nourishing cream, massage cream, essence, eye cream, eye essence, cleansing cream, cleansing foam, cleansing water, a cleansing tissue including the cosmetic composition, a facial pack, a powder, a body lotion, a body cream, a body oil, and a body essence.

In the composition according to one aspect of the present invention, when the formulation of the cosmetic composition is a paste, a cream, or a gel, animal fiber, vegetable fiber, wax, paraffin, starch, tragacanth, cellulose derivative, polyethylene glycol, silicone, bentonite, silica, talc, or zinc oxide, etc. may be used as a carrier component.

In the composition according to one aspect of the present invention, when the formulation of the cosmetic composition is a powder or a spray, lactose, talc, silica, aluminum hydroxide, calcium silicate, or polyamide powder may be used as a carrier component, and particularly in a case of a spray, a propellant such as chlorofluorohydrocarbon, propane/butane, or dimethyl ether may be additionally included.

In the composition according to one aspect of the present invention, when the formulation of the cosmetic composition is a solution or an emulsion, a solvent, a solubilizing agent, or an emulsifying agent is used as a carrier component, such as water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan.

In the composition according to one aspect of the present invention, when the formulation of the cosmetic composition is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth, etc. may be used as a carrier component.

In the composition according to one aspect of the present invention, a functional additive and ingredients included in a general cosmetic composition may be further included in the cosmetic composition. The functional additive may include a component selected from the group consisting of water-soluble vitamins, oil-soluble vitamins, polymeric peptides, polymeric polysaccharides, squalane, sphingolipids, and seaweed extract.

In the composition according to one aspect of the present invention, in addition to the functional additive, ingredients included in a general cosmetic composition may be blended into the cosmetic composition as necessary. As other blending ingredients included, fat and oil components, moisturizers, emollients, emulsifiers, organic and inorganic pigments, organic powders, ultraviolet absorbers, antiseptics, bactericides, antioxidants, plant extracts, pH adjusters, alcohols, colorants, fragrances, blood circulation promoters, cooling agents, antiperspirants, purified water, and the like may be mentioned.

In another aspect, the present invention may relate to a method for exfoliation, skin moisturizing, skin wound healing, promoting skin regeneration, or skin protection against irritation, comprising applying or administering an allantoin micro-needle crystal to a subject in need of exfoliation, skin moisturizing, skin wound healing, promoting skin regeneration, or skin protection against irritation.

In another aspect, the present invention may relate to a use of an allantoin micro-needle crystal for manufacturing a pharmaceutical composition for exfoliation, skin moisturizing, skin wound healing, promoting skin regeneration, or skin protection against irritation.

In another aspect, the present invention may relate to a use of an allantoin micro-needle crystal for manufacturing a cosmetic composition for exfoliation, skin moisturizing, skin wound healing, promoting skin regeneration, or skin protection against irritation.

In another aspect, the present invention may relate to a use of an allantoin micro-needle crystal for manufacturing a food composition for exfoliation, skin moisturizing, skin wound healing, promoting skin regeneration, or skin protection against irritation.

In another aspect, the present invention may relate to a use of an allantoin micro-needle crystal for exfoliation, skin moisturizing, skin wound healing, promoting skin regeneration, or skin protection against irritation.

The present disclosure is further described by the following embodiments which do not limit the scope of the claims.

Embodiment 1. An allantoin micro-needle crystal having an average length of a long axis of 5 to 500 µm.

Embodiment 2. The allantoin micro-needle crystal of Embodiment 1, wherein a thickness of the allantoin micro-needle crystal is greater than 0 µm and 40 µm or less.

Embodiment 3. The allantoin micro-needle crystal of Embodiment 1 or 2, wherein the allantoin micro-needle crystal is soluble.

Embodiment 4. The allantoin micro-needle crystal of any one of Embodiments 1 to 3, wherein the allantoin micro-needle crystal penetrates into skin when applied to the skin.

Embodiment 5. The allantoin micro-needle crystal of any one of Embodiments 1 to 4, wherein the allantoin micro-needle crystal exists inside a skin stratum corneum when 2 to 36 hours have elapsed after being applied to skin.

Embodiment 6. The allantoin micro-needle crystal of any one of Embodiments 1 to 5, wherein the allantoin micro-needle crystal is dissolved and absorbed in skin when repeatedly rolled 10 times with a weight of 0.5 to 1.0 kg/cm² after being applied to the skin.

Embodiment 7. The allantoin micro-needle crystal of any one of Embodiments 1 to 6, wherein the allantoin micro-needle crystal further comprises betaine.

Embodiment 8. A cosmetic composition comprising the allantoin micro-needle crystal according to any one of Embodiments 1 to 7 as an active ingredient.

Embodiment 9. The cosmetic composition of Embodiment 8, wherein the cosmetic composition is for exfoliation, skin moisturizing, skin wound healing, promoting skin regeneration, or skin protection against irritation.

Embodiment 10. The cosmetic composition of Embodiment 8, wherein the cosmetic composition is for exfoliation, skin moisturizing, improvement of damaged skin, promoting skin regeneration, or skin protection against irritation.

Hereinafter, configurations and effects of the present invention will be described in more detail through Manufacturing Examples, Examples, and Experimental Examples. However, the following Manufacturing Examples, Examples, and Experimental Examples are provided only for the purpose of illustration to help understanding of the present invention, and the category and scope of the present invention are not limited thereby.

### [Manufacturing Example] Manufacturing of allantoin micro-needle crystal

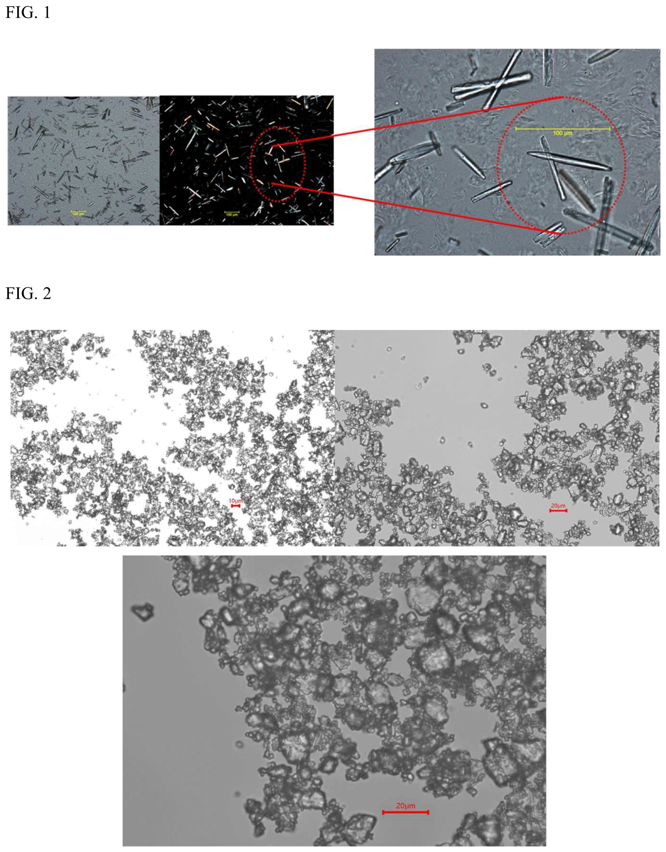

An allantoin micro-needle crystal of Example 1 was manufactured by completely dissolving allantoin in a powder form (allantoin (C₄H₆N₄O₃, molecular weight 158.116 g/mol, IUPAC name (2,5-dioxo-4-imidazolidinyl) urea, CAS No. 97-59-6, EC No. 202-592-8) (3 wt% based on a total weight of the allantoin, polyol, and water combined)) in a polyol (for example, glycerin) using a Deep Eutectic solvent condition through an anti-solvent crystallization method, adding water thereto, and then precipitating the same (FIG. 1). While general allantoin has an atypical shape, as shown in FIG. 1, the allantoin micro-needle crystal of Example 1 has an acicular shape having an average length of a long axis of 150 to 200 µm.

In addition, in the same manner as in Example 1, 6 wt% of allantoin in a powder form based on a total weight of a composition was additionally dissolved in glycerin along with components of Tables 1 and 2 below, and water was added thereto and then precipitated to manufacture Examples 2 to 17. Then, solubility in glycerin, a crystal form, and a sensation when applying them to skin were evaluated and are shown in Tables 1 and 2 below. In this case, as for the solubility, one that does not dissolve was indicated by X, one that slightly dissolves was indicated by △, and one that dissolves well was indicated by O, and the sensation was evaluated by whether a sensation to an extent of receiving a dermatological procedure is felt when Examples 2 to 17 are applied to a face.

**TABLE 1**

| Unit: wt% | | | | | | | | |
|---|---|---|---|---|---|---|---|---|
| | Example 2 | Example 3 | Example 4 | Example 5 | Example 6 | Example 7 | Example 8 | Example 9 |
| Allantoin | 6 | 6 | 6 | 6 | 6 | 6 | 6 | 6 |
| Urea | - | - | - | 2 | 6 | 2 | - | - |
| Betaine | 6 | 12 | 18 | 18 | 18 | 18 | - | - |
| Glycerine | 30 | 30 | 30 | 30 | 30 | 30 | - | 10 |
| Butylene glycol | - | - | - | - | - | 5 | - | - |
| Sodium hyaluronate | - | - | - | - | - | - | - | - |
| Sodium carboxymethyl cellulose | - | - | - | - | - | - | - | - |
| Water | To 100 | To 100 | To 100 | To 100 | To 100 | To 100 | To 100 | To 100 |
| Total Sum | 100 | 100 | 100 | 100 | 100 | 100 | 100 | 100 |
| Solubility | △ | △ | O | O | O | O | △ | △ |
| Form | Acicular | Acicular | Acicular | Acicular | Acicular | Acicular | Atypical + Acicular | Atypical + Acicular |
| Sensation | O | O | O | O | O | O | X | X |

**TABLE 2**

| Unit: wt% | | | | | | | | |
|---|---|---|---|---|---|---|---|---|
| Component | Example 10 | Example 11 | Example 12 | Example 13 | Example 14 | Example 15 | Example 16 | Example 17 |
| Allantoin | 6 | 6 | 6 | 6 | 6 | 6 | 12 | 12 |
| Urea | - | - | - | - | - | - | - | - |
| Betaine | - | 3 | - | - | - | - | 12 | 36 |
| Glycerin | 20 | 30 | 30 | 30 | 30 | 30 | 30 | 30 |
| Sodium hyaluronate | - | - | 1 | 3 | - | - | - | - |
| Sodium carboxymethyl cellulose | - | - | - | - | 3 | 5 | - | - |
| Water | To 100 | To 100 | To 100 | To 100 | To 100 | To 100 | To 100 | To 100 |
| Total Sum | 100 | 100 | 100 | 100 | 100 | 100 | 100 | 100 |
| Solubility | △ | △ | △ | △ | △ | △ | △ | △ |
| Form | Atypical + Acicular | Atypical + Acicular | Atypical + Acicular | Atypical + Acicular | Atypical + Acicular | Atypical + Acicular | Atypical + Acicular | Atypical + Acicular |
| Sensation | X | X | X | X | X | X | X | X |

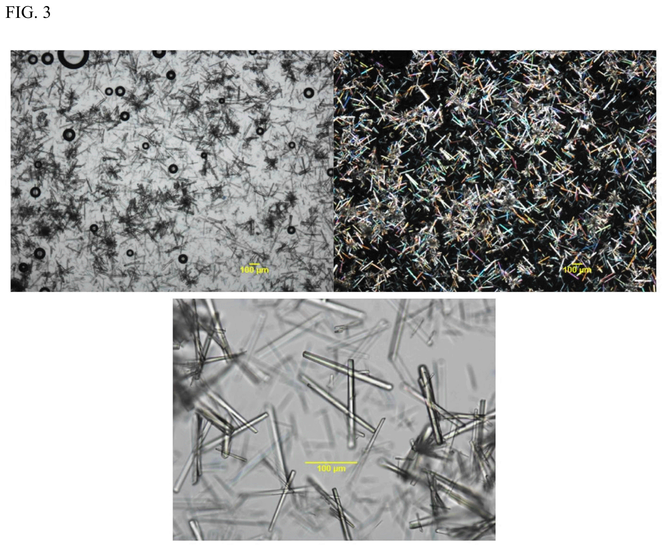

As shown in Tables 1 and 2, it was confirmed that in Examples 2 to 7, atypical allantoin was crystallized into an acicular shape with a purity of 99% or more and a yield of 96% or more. The allantoin micro-needle crystal has an acicular shape having an average length of a long axis of 150 to 200 µm and a thickness of 5 to 40 µm. FIG. 2 is a photograph when a general allantoin powder (Clariant Produkte (Deutschland) GmbH Co., ALLANTOIN Premium) is dissolved in deionized water, showing that general allantoin has an unstandardized shape, whereas FIG. 3 is a photograph when the allantoin micro-needle crystal of Example 5 is dissolved in deionized water, showing that it was crystallized into an acicular shape. Particularly, when allantoin was intended to be utilized as a component of a cosmetic, there was a difficulty due to poorly soluble characteristics of allantoin. As can be seen through Tables 1 and 2 and FIG. 3, Examples 4 to 7 had excellent solubility in deionized water, thereby being able to solve such a problem.

### [Experimental Example 1] Confirmation of skin penetration effect of allantoin micro-needle crystal

In order to confirm whether the allantoin micro-needle crystal manufactured in the Manufacturing Example can penetrate into skin, an *ex vivo* stratum corneum residual evaluation experiment was performed.

After the allantoin micro-needle crystal of Example 5 stained with Nile-red (Sigma Aldrich Co., 72485-100mg) was applied onto an epidermis of an ex vivo skin biopsy tissue (Genoskin Co., NativeSkin^{™} #NSA20), which is a skin biopsy tissue with a diameter of 20 mm collected from an abdomen of a 54-year-old female, a cross-section of the skin biopsy tissue when 18 hours have elapsed was photographed, and an image thereof is as shown in FIG. 4B. As a control group, an allantoin powder (Clariant Produkte (Deutschland) GmbH Co., ALLANTOIN Premium) was applied to a skin biopsy tissue in the same manner and a cross-section thereof was photographed, and an image thereof is as shown in FIG. 4A. In this case, a specific experimental process is as follows.

### Fluorescence Staining

For fluorescence staining, a Nile-red fluorescence staining reagent was dissolved in a DMSO solution to reach a concentration of 1 mg/mL, and to stain the allantoin micro-needle crystal or the allantoin powder, respectively, Nile-red and the raw material powder were mixed at a volume ratio of 5:1000, and then stained by being left at room temperature for 1 hour. Then, samples were prepared by dissolving the allantoin micro-needle crystal or the allantoin powder in DI water to reach a final concentration of 0.2% or 5%.

### Sample Treatment

On the day the skin biopsy tissue arrived, 2 mL of a tissue culture medium was added, and stabilization and culture were performed in an incubator at 37°C and 5% CO₂ for one day. The next day, 20 µL of the allantoin micro-needle crystal stained with Nile-red was dropped onto an epidermis, and then rubbed with a silicone rubber. After being air-dried in air with a thermomixer at 37°C for 1 hour in a clean bench, additional culture was performed for 17 hours in each incubator at 37°C and 5% CO₂. When 18 hours have elapsed after application, the epidermis was washed with deionized water (DI water), and then fixed with a formalin solution. After preparing frozen section slides with the fixed skin tissue, observation was performed with a Confocal laser scanning microscopy (Carl Zeiss Co., model name: LSM980).

### Microscope Measurement Conditions

For observation of a skin tissue cross-section, images for a Nile-red fluorescence signal and a Bright field signal were obtained, three for each group, using a 40X objective lens under conditions of an excitation wavelength of 561 nm and an emission wavelength of 600 to 700 nm.

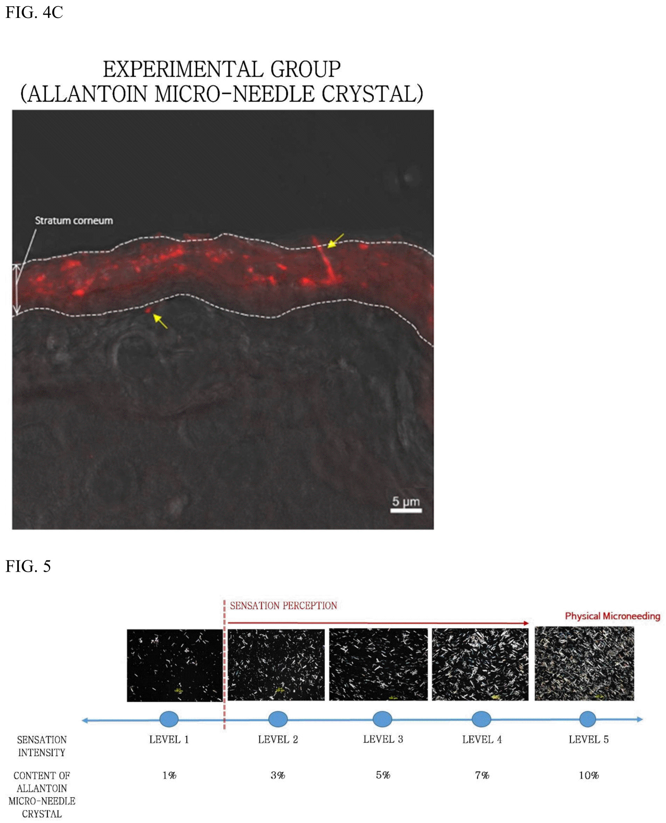

As shown in FIG. 4B, while the allantoin powder, which is a control group, stays at the outermost part of the stratum corneum and thus fails to penetrate skin even after much time has elapsed after application, the allantoin micro-needle crystal of Example 5 remains inside the stratum corneum (FIG. 4C), confirming that the allantoin crystal can be absorbed into skin by easily penetrating into the skin due to its acicular shape.

Through this, it could be seen that the allantoin crystal according to one aspect of the present invention can penetrate into skin and be absorbed into the skin simply by applying it to the skin by having a micro-needle shape like a needle.

### [Experimental Example 2] Confirmation of degree of sensation perception of allantoin micro-needle crystal

The allantoin micro-needle crystal according to one aspect of the present invention is intended to provide an effect similar to easily using a dermatological medical device at home by utilizing a micro-needle technology mainly used in dermatological beauty medical devices. Therefore, it is important to continuously exhibit physical actions and effects similar to receiving a dermatological procedure while being non-invasive when the allantoin micro-needle crystal is applied to skin, and thus a degree of sensation perception according to a content of the allantoin micro-needle crystal was measured.

First, cosmetic compositions of an essence formulation (Comparative Example 1 and Examples 18 to 21) including the allantoin micro-needle crystal of Example 5 were manufactured with components and contents as shown in Table 3 below, and a sensation perception experiment was performed on five subjects in their 30s. The cosmetic compositions of the essence formulation were manufactured by mixing generally used ingredients (specifically, purified water, butylene glycol, dipropylene glycol, glycerin, propylene glycol, 1,2-hexanediol, caprylic/capric triglyceride, ammonium acryloyldimethyltaurate/VP copolymer, polyglyceryl-6 caprylate, polyglyceryl-4 caprate, hydrogenated lecithin, cholesterol, sodium methyl stearoyl taurate, ethylhexylglycerin, and tocopherol) according to a method generally used in a cosmetic industry. The subjects applied Comparative Example 1 and Examples 18 to 21 on their forearms, performed 10 finger rotation operations with a pressure of about 0.5 to 1.0 kg/cm², and then recorded their sensation as scores according to a 5-point scale method in Table 4 below (no physical effect felt: 1 point, weakly felt: 2 points, moderately felt: 3 points, strongly felt: 4 points, very strongly felt: 5 points). After all subjects completed the evaluation, scores recorded by individual subjects were collected, and a sensation perception for each content was confirmed by analyzing a degree of sensation based on the evaluated scores, and results thereof are as shown in Table 5 below.

**TABLE 3**

| Unit: wt% | | | | | |
|---|---|---|---|---|---|
| | Comparative Example 1 | Example 18 | Example 19 | Example 20 | Example 21 |
| Manufactured essence base | 100 | 97 | 95 | 93 | 90 |
| Allantoin micro-needle crystal of Example 5 | 0 | 3 | 5 | 7 | 10 |

**TABLE 4**

| Item | 1 point | 2 points | 3 points | 4 points | 5 points |
|---|---|---|---|---|---|
| Sensation on skin | Not felt at all | Hardly felt | Moderately felt | Strongly felt | Very strongly felt |
| Fine stinging sensation | Not felt at all | Hardly felt | Moderately felt | Strongly felt | Very strongly felt |
| Redness after use | Not red at all | Slightly red | Moderate level | Much red | Very much red |
| Residual sensation after use | None remaining at all | Hardly remaining | Moderate level | Much remaining | Very much remaining |

**TABLE 5**

| Example | Subject | Sensation on skin | Fine stinging sensation | Redness after use | Residual sensation after use |
|---|---|---|---|---|---|
| Comparative Example 1 | Subject 1 | 2 | 2 | 1 | 1 |
| | Subject 2 | 2 | 2 | 1 | 2 |
| | Subject 3 | 1 | 1 | 1 | 1 |
| | Subject 4 | 2 | 2 | 1 | 1 |
| | Subject 5 | 2 | 1 | 1 | 1 |
| | Average | 1.8 | 1.6 | 1 | 1.2 |
| Example 18 | Subject 1 | 3 | 3 | 2 | 2 |
| | Subject 2 | 2 | 3 | 1 | 2 |
| | Subject 3 | 2 | 2 | 1 | 1 |
| | Subject 4 | 2 | 2 | 1 | 1 |
| | Subject 5 | 2 | 1 | 1 | 1 |
| | Average | 2.2 | 2.2 | 1.2 | 1.4 |
| Example 19 | Subject 1 | 3 | 3 | 2 | 2 |
| | Subject 2 | 2 | 3 | 1 | 2 |
| | Subject 3 | 2 | 3 | 1 | 2 |
| | Subject 4 | 2 | 3 | 1 | 2 |
| | Subject 5 | 3 | 3 | 2 | 2 |
| | Average | 2.4 | 3 | 1.4 | 2 |
| Example 20 | Subject 1 | 3 | 4 | 3 | 3 |
| | Subject 2 | 3 | 3 | 1 | 3 |
| | Subject 3 | 3 | 3 | 2 | 2 |
| | Subject 4 | 3 | 3 | 1 | 2 |
| | Subject 5 | 3 | 3 | 2 | 2 |
| | Average | 3 | 3.2 | 1.8 | 2.4 |
| Example 21 | Subject 1 | 4 | 4 | 3 | 4 |
| | Subject 2 | 3 | 4 | 2 | 4 |
| | Subject 3 | 3 | 4 | 2 | 2 |
| | Subject 4 | 3 | 3 | 2 | 3 |
| | Subject 5 | 3 | 3 | 2 | 3 |
| | Average | 3.2 | 3.6 | 2.2 | 3.2 |

As shown in Table 5, as a content of the allantoin micro-needle crystal increased, the sensation perception of most subjects increased. The degree of sensation perception according to the content of the allantoin micro-needle crystal is shown in FIG. 5. Particularly, at 3 wt% or more, most subjects could feel a sensation on skin and a fine stinging sensation, and while they felt the sensation perception effect between the fine stinging sensation and the sensation on skin, the degree of redness after use was not so high (the average value was around 2). In addition, in the residual sensation after use index, most subjects hardly felt a residual sensation as the micro-needle crystals were dissolved and disappeared after 10 rollings. Through this, it could be seen that the allantoin micro-needle crystal according to one aspect of the present invention and a composition including the same provide a sensation perception effect similar to receiving a dermatological procedure by having a shape like a micro-needle, while having no stinging effect to an extent that skin becomes red or painful, and can be used even on sensitive skin as there is no residual sensation because the allantoin micro-needle crystal is dissolved.

### [Experimental Example 3] Confirmation of safety of allantoin acicular crystal

As can be seen from the Experimental Example 2, the allantoin micro-needle crystal according to one aspect of the present invention provides a stinging effect on skin when applied to the skin, and thus the following safety confirmation experiment was performed to confirm safety thereof.

In the same manner as in the Experimental Example 2, the allantoin micro-needle crystal of Example 5 (diluted to a concentration of 5 (w/w %) in distilled water) and a composition of an essence formulation including the allantoin micro-needle crystal of Example 5 (a content of the allantoin micro-needle crystal is 5 wt% based on a total weight of the composition) (Example 22) were manufactured. Then, a closed patch (Van der Bend) test was performed on 30 adult females (average age of 45.1 years) on an upper back for 24 hours and 48 hours to evaluate irritation scores, and average values thereof are shown in Table 6 below.

**TABLE 6**

| Unit: Points | | | | | | | | | |
|---|---|---|---|---|---|---|---|---|---|
| Test substance | Score after 24 hours | | | | Score after 48 hours | | | | Irritation score (Total) |
| | 1+ | 2+ | 3+ | 4+ | 1+ | 2+ | 3+ | 4+ | |
| Example 5 | 2 | 0 | 0 | 0 | 1 | 0 | 0 | 0 | 1.25 |
| Example 22 | 0 | 0 | 0 | 0 | 0 | 0 | 0 | 0 | 0.0 |

As shown in Table 6, the irritation score of the allantoin micro-needle crystal itself of Example 5 was 1.25, which was non-irritating, and the irritation score of the composition including the allantoin micro-needle crystal of Example 22 was 0.0, which was non-irritating.

Through this, it could be seen that the allantoin micro-needle crystal according to one aspect of the present invention and the composition including the same provide a sensation perception effect similar to receiving a dermatological procedure, but are safe enough to be used even on sensitive skin as they are not irritating to skin.

### [Experimental Example 4] Confirmation of stability of allantoin acicular crystal

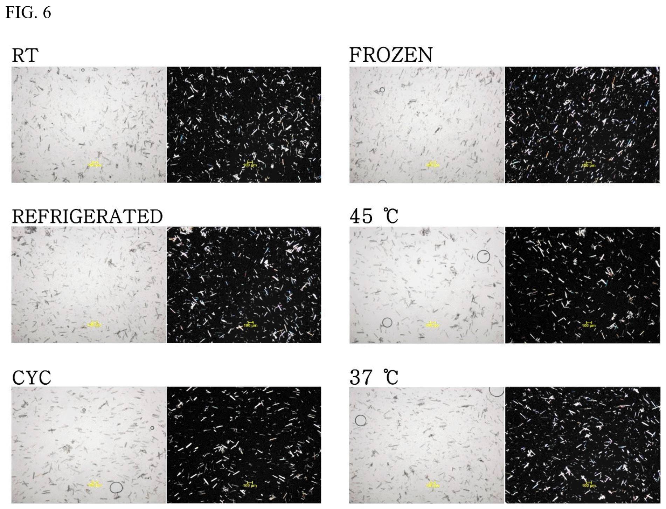

Formulation stability of the allantoin micro-needle crystal manufactured in the Manufacturing Example was confirmed under room temperature (RT), frozen, refrigerated, 37°C, 45°C, and cycling (CYCLE) conditions, respectively. The cycling (CYCLE) condition results show formulation stability confirmed while cycling between -10°C and 45°C every 12 hours. The sample used in this case is the allantoin micro-needle crystal of Example 5. Each sample was taken out from each of the six temperature ranges for a total of 4 weeks and allowed to reach temperature equilibrium at room temperature for 4 hours; thereafter, after confirming the physical properties of the contents with the naked eye, the presence or absence of physical property changes and dissolution of the allantoin micro-needle crystal were confirmed through an electron microscope, and results thereof are as shown in FIG. 6.

As shown in FIG. 6, it could be seen that the allantoin micro-needle crystal according to one aspect of the present invention was stably maintained without a change in physical property even when stored for 4 weeks under harsh conditions.

Therefore, the allantoin micro-needle crystal according to one aspect of the present invention is one in which poorly soluble allantoin is crystallized to have excellent solubility and thus can be utilized as a component of a cosmetic composition, and by having an acicular shape like a needle with an average length of a long axis of 5 to 500 µm, specifically 150 to 200 µm, it can penetrate into skin and be absorbed into the skin simply by applying it to the skin, has excellent safety by being dissolved in the skin through rolling after application, is easily stored and distributed by being stably maintained without a change in physical property even under harsh conditions, and has an excellent effect of being utilizable as an active ingredient delivery system for delivering various components, specifically oil-soluble or water-soluble active ingredients, into the skin.

Hereinafter, formulation examples of a composition according to the present invention will be described, but this is not intended to limit the present invention, but is merely intended to describe the same in detail.

### [Formulation Example 1] Nourishing Cosmetic Water

Nourishing cosmetic water may be manufactured by a conventional method according to a composition described in Table 7 below.

**TABLE 7**

| Name of Raw material | Content (wt%) |
|---|---|
| Glycerin | 3.0 |
| Butylene glycol | 3.0 |
| Propylene glycol | 3.0 |
| Carboxyvinyl polymer | 0.1 |
| Allantoin micro-needle crystal of Example 5 | 0.1 |
| Beeswax | 4.0 |
| Polysorbate 60 | 1.5 |
| Caprylic/Capric triglyceride | 5.0 |
| Squalane | 5.0 |
| Sorbitan sesquioleate | 1.5 |
| Cetearyl alcohol | 1.0 |
| Triethanolamine | 0.2 |
| Antiseptic, fragrance | q.s. |
| Purified water | balance |
| Total | 100 |

### [Formulation Example 2] Nourishing Lotion

Nourishing lotion may be manufactured by a conventional method according to a composition described in Table 8 below.

**TABLE 8**

| Name of Raw material | Content (wt%) |
|---|---|
| Purified water | balance |
| Glycerin | 3.0 |
| Butylene glycol | 3.0 |
| Liquid paraffin | 5.0 |
| Beta-glucan | 7.0 |
| Carbomer | 0.1 |
| Allantoin micro-needle crystal of Example 5 | 10.0 |
| Caprylic/Capric triglyceride | 3.0 |
| Squalane | 5.0 |
| Cetearyl glucoside | 1.5 |
| Sorbitan stearate | 0.4 |
| Polysorbate 60 | 1.5 |
| Antiseptic | q.s. |
| Fragrance | q.s. |
| Colorant | q.s. |
| Triethanolamine | 0.1 |
| Total | 100 |

### [Formulation Example 3] Nourishing Cream

A nourishing cream may be manufactured in a conventional method according to the composition described in Table 9 below.

**TABLE 9**

| Name of Raw material | Content (wt%) |
|---|---|
| Glycerin | 3.5 |
| Butylene glycol | 3.0 |
| Liquid paraffin | 7.0 |
| Beta-glucan | 7.0 |
| Carbomer | 0.1 |
| Allantoin micro-needle crystal of Example 5 | 10.0 |
| Caprylic/Capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Antiseptic, fragrance | q.s. |
| Purified water | balance |
| Total | 100 |

### [Formulation Example 4] Ointment

Ointment may be manufactured by a conventional method according to a composition described in Table 10 below.

**TABLE 10**

| Name of Raw material | Content (wt%) |
|---|---|
| Glycerin | 8.0 |
| Butylene glycol | 4.0 |
| Liquid paraffin | 15.0 |
| Beta-glucan | 7.0 |
| Carbomer | 0.1 |
| Allantoin micro-needle crystal of Example 5 | 0.1 |
| Caprylic/Capric triglyceride | 3.0 |
| Squalane | 1.0 |
| Cetearyl glucoside | 1.5 |
| Sorbitan stearate | 0.4 |
| Cetearyl alcohol | 1.0 |
| Antiseptic | q.s. |
| Fragrance | q.s. |
| Colorant | q.s. |
| Beeswax | 4.0 |
| Purified water | balance |

In view of the foregoing, specific portions of the content of the present invention have been described in detail, and to a person having ordinary skill in the art, such specific descriptions are merely a preferable embodiment, and it will be apparent that the scope of the present invention is not limited thereby. Therefore, the substantial scope of the present invention is defined by the appended claims and their equivalents.

## Claims

1. An allantoin micro-needle crystal having an average length of a long axis of 5 to 500 µm.

2. The allantoin micro-needle crystal of claim 1, wherein a thickness of the allantoin micro-needle crystal is greater than 0 µm and 40 µm or less.

3. The allantoin micro-needle crystal of claim 1 or 2, wherein the allantoin micro-needle crystal is soluble.

4. The allantoin micro-needle crystal of any one of claims 1 to 3, wherein the allantoin micro-needle crystal penetrates into skin when applied to the skin.

5. The allantoin micro-needle crystal of any one of claims 1 to 4, wherein the allantoin micro-needle crystal exists inside a skin stratum corneum when 2 to 36 hours have elapsed after being applied to skin.

6. The allantoin micro-needle crystal of any one of claims 1 to 5, wherein the allantoin micro-needle crystal is dissolved and absorbed in skin when repeatedly rolled 10 times with a weight of 0.5 to 1.0 kg/cm² after being applied to the skin.

7. The allantoin micro-needle crystal of any one of claims 1 to 6, wherein the allantoin micro-needle crystal further comprises betaine.

8. A cosmetic composition comprising the allantoin micro-needle crystal according to any one of claims 1 to 7 as an active ingredient.

9. The allantoin micro-needle crystal of any one of claims 1 to 7 for use in skin wound healing.

10. A use of the allantoin micro-needle crystal according to any one of claims 1 to 7 for exfoliation, skin moisturizing, skin wound healing, promoting skin regeneration, or skin protection against irritation.
